**Europäisches Patentamt**

**European Patent Office**

**Office européen des brevets**

(19)

(11) Veröffentlichungsnummer: **0 210 204**
**B1**

(12) **EUROPÄISCHE PATENTSCHRIFT**

(45) Veröffentlichungstag der Patentschrift:
**07.12.88**

(21) Anmeldenummer: **86900765.8**

(22) Anmeldetag: **20.01.86**

(86) Internationale Anmeldenummer:
**PCT/EP 86/00016**

(87) Internationale Veröffentlichungsnummer:
**WO 86/04217 (31.07.86 Gazette 86/17)**

(51) Int. Cl.⁴: **A 23 J 3/00, A 61 K 37/18,**
**A 61 K 37/02, C 12 P 9/00,**
**C 12 N 9/00**

┌─────────────────┐
│ E R R A T U M │
└─────────────────┘

(SEITE, SPALTE, ZEILE)
(PAGE, COLUMN, LINE)
(PAGE, COLONNE, LIGNE)

| DIE TEXTSTELLE : TEXT PUBLISHED : LE PASSAGE SUIVANT : | | | | LAUTET BERICHTIGT : SHOULD READ : DEVRAIT ETRE LU : |
|---|---|---|---|---|
| gemäss Beispiel hydroly- | 5 | 8 | 12 | gemäss Beispiel 6 hydroly- |
| siert und wie in Beispiel | 5 | 8 | 13 | siert und wie in Beispiel 6 |

| Tag der Entscheidung über die Berichtigung Date of decision on rectification: Date de décision portant sur modification: | ) ) ) 06.03.89 ) ) | Ausgabe- und Veröffentlichungstag: Issue and publication date: Date d'edition et de publication: | ) ) ) 03.05.89 ) ) | Patbl.Nr) EPB no:) .89/.18 Bull. no:) |

**Europäisches Patentamt**

**European Patent Office**

**Office européen des brevets**

(19)

(11) Veröffentlichungsnummer: **0 210 204 B1**

(12) # EUROPÄISCHE PATENTSCHRIFT

(45) Veröffentlichungstag der Patentschrift:
**07.12.88**

(21) Anmeldenummer: **86900765.8**

(22) Anmeldetag: **20.01.86**

(86) Internationale Anmeldenummer:
**PCT/EP 86/00016**

(87) Internationale Veröffentlichungsnummer:
**WO 86/04217 (31.07.86 Gazette 86/17)**

(51) Int. Cl.⁴: **A 23 J 3/00**, A 61 K 37/18, A 61 K 37/02, C 12 P 9/00, C 12 N 9/00

(54) **PROTEINHYDROLYSATE, VERFAHREN ZU IHRER HERSTELLUNG UND ARZNEIMITTEL, DIE DIESE HYDROLYSATE ENTHALTEN.**

(30) Priorität: **18.01.85 DE 3501560**
**24.05.85 DE 3518828**

(43) Veröffentlichungstag der Anmeldung:
**04.02.87 Patentblatt 87/6**

(45) Bekanntmachung des Hinweises auf die Patenterteilung:
**07.12.88 Patentblatt 88/49**

(84) Benannte Vertragsstaaten:
**AT BE CH DE FR GB IT LI LU NL SE**

(56) Entgegenhaltungen:
**EP-A- 0 106 309**

(73) Patentinhaber: **Gauri, Kailash Kumar, Dr. Prof., Zur Waldburg 13, D-2359 Lentföhrden (DE)**

(72) Erfinder: **Gauri, Kailash Kumar, Dr. Prof., Zur Waldburg 13, D-2359 Lentföhrden (DE)**

(74) Vertreter: **Kinzebach, Werner, Dr., Patentanwälte Reitstötter, Kinzebach und Partner Sternwartstrasse 4 Postfach 86 06 49, D-8000 München 86 (DE)**

## Beschreibung

Die Erfindung betrifft ein Proteinhydrolysat, das aus Molkenprotein, Lactalbumin, α-Lactalbumin, Lactoferrin, β-Lactoglobulin, Lysozym oder Serumalbumin gewonnen wird, ein Verfahren zur Herstellung dieses Hydrolysates und seine Verwendung für pharmazeutische Zwecke.

Bekanntlich setzen sich die Proteine der Milch zusammen aus den Caseinen und Molkenproteinen. Ausserdem können die Proteose-Peptone mit einem Anteil am Gesamtstickstoffgehalt der Kuhmilch von etwa 5% zu den Proteinen gerechnet werden. Der Anteil der Caseine am Gesamt-Proteingehalt der Kuhmilch beträgt etwa 80%, der an Molken-Proteinen etwa 20%. Die Caseine gehören zu den am besten erforschten Proteinen. Im Gegensatz zu den ursprünglichen Annahmen sind sie keineswegs einheitliche Körper. Bisher sind mehrere Fraktionen unterschieden worden. Typisch für alle Caseine ist jedoch, dass sie gebundenen Phosphor enthalten. Sie sind sehr viel komplexer aufgebaut und haben ein wesentlich grösseres Molekulargewicht als die sogenannten Molken-Proteine. Von den Molken-Proteinen ist bisher bekannt, dass sie aus β-Lactoglobulin, α-Lactalbumin, Serumalbumin und Immunglobulinen bestehen.

Untersuchungen von Brantl und Teschemacher (Milchwissenschaft 37, Seiten 641-644, 1982) haben gezeigt, dass opiatartig wirkende Stoffe aus der Caseinfraktion von Rindermilch isoliert werden können. Ausgehend von käuflichem Casein-Pepton haben diese Autoren einen Chloroform-Methanol 65:35 (v/v) Extrakt angefertigt und diesen über zwei Adsorptions-/Desorptionsschritte an Aktivkohle bzw. XAD-2-Polystyrolharz vorgereinigt und mittels HPLC weitergereinigt. Die Opiataktivität des jeweils erhaltenen Materials wurde am Meerschweinchen-Ileum-Präparat bestimmt und so die Anreicherung bzw. Reinigung quantitativ verfolgt. Die opiatartige Wirkung wurde einem Heptapeptid und seinen am C-Terminus um eine oder mehrere Aminosäurereste verkürzten Fragmenten zugeordnet.

Der Erfindung liegt die Aufgabe zugrunde, aus bestimmten anderen Proteinen weitere pharmazeutisch brauchbare Bestandteile bereitzustellen.

Überraschenderweise wurde nun gefunden, dass ausgehend von Molkenprotein, Lactalbumin, α-Lactalbumin, Lactoferrin, β-Lactoglobulin, Lysozym oder Serumalbumin Abbauprodukte gewonnen werden können, die überraschende pharmakologische Wirkungen besitzen.

Insbesondere Abbauprodukte von Lactalbumin, α-Lactalbumin, β-Lactoglobulin, Lactoferrin oder dem Gemisch aller Molkenproteine sowie von denaturiertem Lysozym zeigten überraschende pharmakologische Wirkungen. Aufgrund der oben erwähnten Arbeiten war man bisher der Auffassung, dass pharmakologisch wirksame Bestandteile der Milch nur in der Casein-Fraktion vorkommen. Lysozym ist in seinem Aufbau dem Lactalbumin ähnlich. In beiden Proteinen sind 51 Aminosäuren identisch und 24 ähnlich angeordnet. Lysozym kommt in geringer Menge in der Kuhmilch vor, in grösserer Menge in Hühnereiweiss und in Schleimhäuten.

Das erfindungsgemässe Proteinhydrolysat ist dadurch erhältlich, dass man aus Molkenprotein, Lactalbumin, Lactoferrin, α-Lactalbumin, β-Lactoglobulin, Lysozym oder Serumalbumin und Wasser eine etwa 1 bis 10 gew.%ige Suspension herstellt, diese bei 35 bis 38°C mehrere Stunden unter Rühren mit mindestens einer Protease und gegebenenfalls einer Lipase behandelt, diese Behandlung bei etwa 60°C einige Stunden fortsetzt, auf 80 bis 90°C erwärmt, bei dieser Temperatur kurz verweilt, dann abkühlen lässt, nach dem Abkühlen unter reduziertem Druck einengt und den Rückstand als Produkt (A) isoliert; letzteren Rückstand mit einem polaren Lösungsmittel oder Lösungsmittelgemisch bei Raumtemperatur oder leicht erhöhter Temperatur extrahiert, filtriert und aus dem Filtrat durch Einengen zur Trockene das Produkt (AP) gewinnt.

Vorteilhaft verwendet man eine 2 bis 5 gew.%ige Suspension des Ausgangsmaterials in Wasser.

Vorzugsweise dauert die Behandlung bei 35 bis 38°C etwa 1 bis 4 Stunden, vorteilhaft etwa 2 Stunden und die erhöhte Temperatur von etwa 60°C wird vorteilhaft etwa 1 bis 4, vorzugsweise etwa 2 bis 3 Stunden beibehalten.

Als polares Lösungsmittel verwendet man vorzugsweise absolutes Äthanol, Chloroform oder Isopropanol und als polares Lösungsmittelgemisch empfiehlt sich ein Chloroform/Methanol-Gemisch (1/1 bis 3/1 Vol./Vol.) oder ein Äthanol-Wasser-Gemisch mit 20 bis 80% Wasser zu verwenden.

Als Protease(n) verwendet man vorzugsweise Papain, Pankreatin, Chymotrypsin, Trypsin und gegebenenfalls eine aus Pilzen und/oder Bakterien gewonnene Protease, wobei die Pilzprotease ausgewählt wird unter den Proteasen aus Tritirachium-Arten, insbesondere Tritirachium alba, Proteasen aus Aspergillus-Arten, insbesondere Aspergillus saitoi, Aspergillus sojae, Aspergillus oryzae und/oder aus Rhizopus-Arten, insbesondere Newlase, und wobei die Bakterienprotease ausgewählt wird unter Proteasen aus Streptomyces-Arten, insbesondere Streptomyces caespitosus, Streptomyces griseus (Pronase E), aus Bacillus subtilis-Arten, insbesondere Subtilopeptidase A (Carlsberg Subtilisin), und aus Bacillus polymyxa.

Bei einem Ausgangsmaterial, das noch merkliche Beimengungen an Stärke und stärkeähnlichen Produkten enthält, ist es vorteilhaft, eine α-Amylase aus einer Subtilis-Art mitzuverwenden. Das Aktivitätsoptimum einer derartigen Amylase liegt in der Regel bei einem pH-Wert von 5,7 bis 7,2 und mit der Temperatur kann man bis zu 75°C gehen.

Die Mitverwendung einer Lipase ist in der Regel auch nur dann erforderlich, wenn das Ausgangsmaterial merkliche Beimengungen an Fetten enthält.

Vorteilhaft werden die Enzyme in einer Menge von etwa 0,01 bis 2 Gew.%, bezogen auf die Suspension, verwendet.

Als Protease verwendet man vorzugsweise Papain oder ein Gemisch von etwa gleichen Gewichtsteilen Pankreatin und Papain.

Ein weiteres vorteilhaftes Proteasengemisch ist ein Gemisch aus etwa gleichen Anteilen Papain, Pankreatin und einer Bakterien- oder Pilzprotease, z.B. die Bakterienprotease Pronase E aus Streptomyces griseus und als Pilzprotease das Produkt «Newlase» aus einer Aspergillus-Art.

Vorzugsweise werden die wärmebeständigen Enzyme in der zweiten Erwärmungsphase zugegeben, das heisst nach dem ersten Erwärmen auf 35 bis 38°C.

Das oben erwähnte Produkt (AP) kann weiter gereinigt und aufgetrennt werden, indem man dieses Produkt in der etwa 10 bis 20fachen Gewichtsmenge eines 40 bis 80%igen wässrigen Äthanols suspendiert, mit etwa der gleichen Volumenmenge eines aliphatischen oder zykloaliphatischen Kohlenwasserstoffes mit etwa 4 bis 8 C-Atomen wiederholt extrahiert, aus den einzelnen Phasen (der wässrig alkoholischen und dem Kohlenwasserstoffextrakt) jeweils das Lösungsmittel entfernt, wobei man als Rückstand der wässrig alkoholischen Phase das Produkt (A$_2$) und als Rückstand des Kohlenwasserstoffextraktes das Produkt (P) erhält. Das Produkt (A$_2$) wird vorteilhaft mit der etwa 5 bis 20fachen Gewichtsmenge Diäthyläther extrahiert, der dabei ungelöst zurückbleibende Anteil wird mit der etwa 20 bis 40fachen Gewichtsmenge Chloroform intensiv gerührt, anschliessend filtriert, das Filtrat zur Trockene eingeengt und damit das Produkt (F) isoliert. Der chloroformunlösliche Anteil ist das Produkt (N).

Zur Erhöhung der Ausbeute kann vorteilhafterweise der bei der Behandlung des Produktes (A$_2$) anfallende Ätherextrakt eingeengt werden und der dabei erhaltene Rückstand mit dem oben erwähnten Produkt (P) vereinigt werden. Letzteres kann zur weiteren Reinigung vorteilhafter Weise in absolutem Äthanol gelöst, bis zu einer beständigen Trübung mit H$_2$O versetzt werden und danach mit einem aliphatischen oder cycloaliphatischen Kohlenwasserstoff mit etwa 4 bis 8 C-Atomen extrahiert werden.

Nach dem Entfernen des Extraktionsmittels erhält man gereinigtes Produkt (P), das bei Raumtemperatur fest ist, oberhalb 30°C jedoch flüssig wird.

Vorteilhafterweise kann aus der wässrig-äthanolischen Phase durch Einengen weiteres Produkt (A$_2$) gewonnen werden, das wie oben bereits beschrieben, weiterverarbeitet werden kann und bei der beschriebenen Auftrennung überwiegend das Produkt (F) ergibt.

Die oben beschriebenen Produkte (F) und (N) können Chloroform-Reste enthalten, diese können entfernt werden, indem man die Produkte (F) und (N) in wenig Äthanol löst und das Äthanol im Vakuum abzieht.

Bei den oben erwähnten aliphatischen und cycloaliphatischen Wasserstoffen mit 4 bis 8 C-Atomen handelt es sich vorzugsweise um n-Hexan, Cyclohexan, Heptan, Octan oder Petroläther mit einem Siedebereich von etwa 40 bis 70°C.

Die erfindungsgemäss erhältlichen Proteinhydrolysate weisen wertvolle pharmakologische Eigenschaften auf. Sie wirken z.B. analgetisch, antiphlogistisch, antimutagen und haben eine Antiglaucomwirkung. Sie können daher z.B. zur Behandlung von schmerzhaften Entzündungen aller Art, Neurodermitis, Arthritis, Rheuma aber auch Glaucoma verwendet werden. Die erfindungsgemässen Produkte (AB) und (A) wirken vorwiegend analgetisch, antimutagen und antiphlogistisch. Das erfindungsgemässe Produkt F ist besonders durch seine antiphlogistische und Antiglaucoma-Wirkung gekennzeichnet.

Das erfindungsgemässe Produkt N wirkt hauptsächlich analgetisch und antimutagen.

Das erfindungsgemässe Produkt P eignet sich vorteilhaft als Salbengrundlage.

Das aus Lactoferrin gewonnene erfindungsgemässe Produkt (N) zeigt eine besonders ausgeprägte antimutagene Wirkung.

Die Verwendung der erfindungsgemässen Proteinhydrolysate bei der prophylaktischen und therapeutischen Behandlung der oben genannten Krankheitsbilder ist daher vorteilhaft.

Die Erfindung betrifft auch pharmazeutische Zusammensetzungen, welche wenigstens eines der erfindungsgemässen Proteinhydrolysate, gegebenenfalls zusammen mit einem für pharmazeutische Zwecke geeigneten Träger und/oder Hilfsstoff, enthalten.

Diese Zusammensetzungen können insbesondere bei den oben angegebenen Indikationen Verwendung finden, sie können z.B. oral, parenteral oder topisch angewendet werden. Die Dosierung hängt in erster Linie von der spezifischen Verarbeitungsform und dem Zweck der Therapie bzw. Prophylaxe ab.

Bei oraler Verabreichung liegt die Einzeldosis im allgemeinen zwischen 0,5 und 50 mg (für einen erwachsenen Menschen mit einem Körpergewicht von etwa 70-75 kg) und es werden etwa 3-10 Dosen pro Tag (24 Stunden) verabreicht. Bei der Behandlung von Neurodermitis kann man sich im unteren Dosierungsbereich bewegen, z.B. bei 1,5-3 mg Wirkstoff pro Einzeldosis. Bei dieser Dosierung verschwindet der Juckreiz rasch und anschliessend normalisiert sich die Haut. Bei der Behandlung von Rheuma kann die Tagesdosis bis zu 500 mg (bei einem erwachsenen Menschen) betragen.

Bei intravenöser Verabreichung werden in der Regel 70-140 mg pro Person (von etwa 75 kg Körpergewicht) pro Tag verabreicht. In der Regel wird diese Dosis auf einmal pro Tag verabreicht.

Bei topischer Anwendung wird in der Regel das 1- bis 2fache der oben für die orale Anwendung angegebenen Wirkstoffmenge verabreicht.

Ein Präparat für die orale Anwendung kann als Lösung, z.B. in Wasser oder Alkohol oder als Tablette formuliert sein, wobei für die Tablettenherstellung die üblichen physiologisch unbedenklichen Füll-, Binde-, Spreng- und Gleitmittel verwendet werden können. Geeignete Füllstoffe sind z.B. Milchzucker, Rohrzucker, Stärke oder Cellulose und ihre Derivate. Brauchbare Bindemittel sind z.B. Stärke, Gelatine, Zucker, Celluloseäther, Polymere, z.B. Polyvinylpyrrolidon. Als Sprengmittel können auch Stärke und Stärkeäther verwendet werden. Geeignete Gleit- und Formtrennmittel sind z.B. Talkum, Stearate oder Silikone und als Fliessreguliermittel kann man hochdisperses Siliciumdioxid oder Talkum verwenden. Die Tabletten können auch als überzogene Tabletten oder als Filmtabletten formuliert sein. Natürlich kann die Zubereitung auch in einer üblichen Weichgelatine- oder Hartgelatinekapsel verabreicht werden.

Für Injektionszwecke können übliche sterile, isotonische wässerige Lösungen hergestellt werden. Der Wirkstoff kann aber auch als Lyophilisat bereitgehalten werden, das vor der Anwendung in einem geeigneten wässerigen Verdünnungsmittel aufgelöst wird.

Ein Präparat für die topische Anwendung kann als wässerige Lösung, Lotion, Gelee, ölige Lösung, Suspension, fetthaltige oder Emulsions-Salbe vorliegen. Ein Präparat in Form einer wässerigen Lösung erhält man beispielsweise dadurch, dass man die erfindungsgemässen Wirkstoffe in einer wässerigen Pufferlösung von pH 4 bis 7,5 löst und gewünschtenfalls einen weiteren Wirkstoff und/oder ein polymeres Haftmittel, z.B. Polyvinylpyrrolidon, und/oder ein Konservierungsmittel zufügt. Die Konzentration des Wirkstoffs beträgt etwa 1 bis 10 Gew.%.

Eine ölige Applikationsform für die topische Verabreichung erhält man beispielsweise durch Suspendieren der erfindungsgemässen Wirkstoffe in einem Öl, gegebenenfalls unter Zusatz von Quellmitteln, wie Aluminiumstearat, und/oder grenzflächenaktiven Mitteln (Tensiden), deren HLB-Wert (hydrophilic-lipophilic-balance) unter 10 liegt, wie Fettsäuremonoester mehrwertiger Alkohole, z.B. Glycerinmonostearat, Sorbitanmonolaurat, Sorbitanmonostearat oder Sorbitanmonooleat.

Eine fetthaltige Salbe erhält man z.B. durch Suspendieren der erfindungsgemässen Wirkstoffe in einer streichbaren Fettgrundlage, gegebenenfalls unter Zusatz eines Tensids mit einem HLB-Wert unter 10.

Eine vorteilhafte Salbengrundlage ist das erfindungsgemässe Produkt (P).

Die antimutagene Wirkung wurde mit Hilfe des Sister-Chromatid-Exchange-Tests an chinesischen Hamstern nachgewiesen, diese Methode haben H. Marquardt und U. Bayer in Mutation Research 56, 169-176 (1977) beschrieben.

Die lokale Analgesie wurde mit dem sogenannten Frey'schen-Haar an der Augenhornhaut des Kaninchens bestimmt, man vergleiche M.v. Frey, Beiträge zur Physiologie des Schmerzsinnes, Bericht über die Verhandlungen der Königlich sächsischen Gesellschaft der Wissenschaften zu Leipzig, Mathemat. Physisch. Classe Leipzig, Herausgeber Hirzel (1894), 185-196.

Beispiel für die Behandlung von Neurodermitis:

12 Patienten, die an Neurodermitis erkrankt waren und daher am ganzen Körper starken Juckreiz verspürten, wurde jeweils ein Tropfen einer 5%igen Lösung des erfindungsgemässen Produktes (F) in 20%igem wässerigem Äthanol dreimal täglich oral appliziert. Eine zweite Gruppe von 12 Patienten diente als Kontrollgruppe (nur wässeriges Äthanol appliziert). Bei den Patienten der Wirkstoffgruppe war der Juckreiz in etwa 30 Minuten nach der Applikation verschwunden. Nach viertägiger Behandlung war die Hautentzündung stark zurückgegangen.

Je nach eingesetzter Protease erhält man erfindungsgemäss vorwiegend antiphlogistisch wirksame Peptide, welche im allgemeinen nur wenig analgetisch wirksam sind oder einen hohen Anteil an analgetisch wirksamen Peptiden. Während Pankreatin und Papain überwiegend zu entzündungshemmenden Peptiden führen, welche eine Schutzwirkung gegenüber Pentoxyfyllin-Schädigungen aufweisen, erhält man durch die Kombination von Pankreatin und/oder Papain und Proteasen aus Pilz oder Bakterien, Peptide zusätzlich mit analgetischer Wirkung. Mit anderen Worten führen Proteasen, die bei höherer Temperatur arbeiten, beispielsweise solche aus Pilzen und Bakterien, zu einem hohen Anteil an analgetisch wirksamen Peptiden.

Die nachfolgenden Beispiele dienen zur weiteren Erläuterung der Herstellung der erfindungsgemässen Substanzen.

*Beispiel 1*

5 g einer denaturierten Milcheiweissfraktion aus Molke (im Handel als Lactalbumin, z.B. als Lactalbumin-Sigma erhältlich) werden in 95 ml Wasser suspendiert, unter Rühren gibt man 0,4 g eines Gemisches aus jeweils etwa gleichen Gewichtsanteilen Pankreatin, Papain und eine Bakterien- oder Pilzprotease hinzu und erwärmt auf 35 bis 37°C. Bei dieser Temperatur rührt man mehrere Stunden bis das Reaktionsgemisch annähernd klar ist. Sodann wird auf 60°C erhitzt, nach ca. 1 Stunde langsam auf 80°C gesteigert, kurze Zeit bei dieser Temperatur gelassen, abgekühlt und im Vakuum eingeengt. Der Rückstand wird in 100 ml Ethanol/Wasser 70:30 (v/v) aufgenommen, mit etwas Aktivkohle und Celite versetzt, filtriert und das Filtrat wird zur Trockene eingeengt. Man erhält ein farbloses Produkt in etwa 70-80%iger Ausbeute.

*Beispiel 2*

5 g Lactalbumin werden in 130 ml Wasser suspendiert, man gibt 50 mg Papain und 50 ml Pankreatin und 50 mg einer Lipase hinzu, erwärmt auf 35 bis 37°C, rührt bei dieser Temperatur etwa 2 Stunden lang, gibt 50 mg einer handelsüblichen Pilzprotease, z.B. Newlase hinzu und steigert die Temperatur langsam (innerhalb von etwa 2-3 Stunden) auf 60°C. Dann erhöht man die Temperatur kurz auf 80°C, lässt abkühlen, engt die trübe Lösung im Vakuum ein, nimmt den Rückstand in Ethanol auf, filtriert und engt im Vakuum ein. Man erhält etwa 0,2 g Produkt (AP).

*Beispiel 3*

Analog Beispiel 1 werden 5 g Lactalbumin in 130 ml Wasser suspendiert. Man gibt jeweils 50 mg von zwei proteolytischen Enzymen, z.B. Papain und Pankreatin hinzu, erwärmt auf 38°C und rührt etwa 2 Stunden lang bei dieser Temperatur. Anschliessend wird die Temperatur auf 80°C erhöht und gemäss Beispiel 1 aufgearbeitet. Man erhält etwa 1,8 g Produkt.

*Beispiel 4*

Analog Beispiel 1 werden 5 g Lactalbumin in 130 ml Wasser suspendiert. Man gibt 50 mg Papain hinzu, erwärmt auf 35 bis 37°C, rührt eine Stunde lang bei dieser Temperatur, gibt erneut 50 mg Papain hinzu, rührt bei dieser Temperatur etwa 2 Stunden lang weiter, erhitzt kurz auf 80 °C und engt nach dem Abkühlen das Gemisch im Vakuum zur Trockene ein. Behandelt man anschliessend mit Ethanol/Wassser,

wie in Beispiel 1 beschrieben, so erhält man etwa 1,7 g Produkt.

*Beispiel 5*

5 g Lactalbumin werden gemäss den Angaben des Beispiels 4 suspendiert und behandelt jedoch wird anstelle von Papain in diesem Falle Pankreatin verwendet. Nach der Alkohol/Wasser-Behandlung erhält man etwa 2,1 g Produkt.

*Beispiel 6*

5 g Lactoblobulin oder 5 g Lactoferrin werden nach Zusatz von 200 ml Wasser mit einem Gemisch aus jeweils 50 mg Pankreatin, Papain und Pilzprotease wie unter Beispiel 1 beschrieben, hydrolysiert und aufgearbeitet. Nach der Fällung mit 70:30 Ethanol:Wasser erhält man 5,6 g eines Peptidgemisches.

Als Pilzproteasen werden verschiedene Produkte eingesetzt. In einem Ansatz verwendet man als Enzymgemisch ein lyophilisiertes Pulver aus Tritirachium-Arten, insbesondere aus Tritirachium alba mit einem Proteingehalt von ungefähr 90% und einer Aktivität von ungefähr 10 bis 20 E/mg Protein. In einem weiteren Ansatz verwendet man Protease aus Aspergillus-Arten, insbesondere Aspergillus saitoi mit einer Aktivität von ungefähr 0,3 E/mg Trockenmasse bzw. Aspergillus sojae mit gleicher Aktivität bzw. Aspergillus oryzae. In einem weiteren Ansatz verwendet man Protease aus Rhizopus-Arten, mit einer Aktivität von ungefähr 0,5 E/mg Masse. Das Enzym wird auch «Newlase» genannt.

Jeweils bei einer Dosis von 250 mg/kg erzielt man mit dem Lactoglobulin-Peptid-Hydrolysat gegenüber 300 mg/kg i.p. Pentoxyphyllin einen Schutzeffekt.

*Beispiel 7*

Wie in Beispiel 6 beschrieben, wird 1 g β-Lactoglobulin mit Pankreatin, Papain und einer Bakterienprotease (aus Hefe) in einer Menge von jeweils 10 mg hydrolisiert und wie zuvor beschrieben aufgearbeitet.

Die Herstellung des Enzymgemisches erfolgt durch Dekapselierung mit 50% ethoxyliertem Fettalkohol. Es handelt sich um eine Endopeptidase mit einer Aktivität von ungefähr 440 Delfter-Einheiten/mg Trockenmasse. Die Dichte des Proteins beträgt 750 bis 900 g/l. Das Optimum der Aktivität liegt bei pH 7 bis 11 und einer Temperatur von etwa 60°C.

Weitere Ansätze werden hergestellt unter Verwendung von Streptomyces-Arten, insbesondere Streptomyces caespitosus mit einer Aktivität von 0,7 bis 1 E/mg Trockenmasse, mit Subtilis-Arten mit einer Aktivität von 7-15 E/mg Protein, insbesondere dem Enzym Subtilopeptidase A (Carlsberg, Subilisin), mit Streptomyces griseus (Pronase E) mit einer Aktivität von 4 E/mg Trockenmasse, gereinigt, oder Streptomyces griseus mit einer Aktivität von 15 bis 20 E/mg Trockenmasse, oder mit Bacillus Polymyxa mit einer Aktivität von ungefähr 0,4 E/mg Trockenmasse.

*Beispiel 8*

5 g α-Lactalbumin werden wie in Beispiel 6 beschrieben hydrolysiert und aufgearbeitet. Die Ausbeute beträgt 5,5 g.

*Beispiel 9*

100 g α-Lactalbumin werden gemäss Beispiel 6 hydrolisiert. Bei der Aufarbeitung wird anstelle des Ethanol/Wasser-Gemisches absolutes Ethanol verwendet. Die Ausbeute beträgt ca. 2,5 g. Das Peptidgemisch zeigt eine lokale analgetische Wirkung bei Konzentrationen zwischen 0,3 und 3%.

*Beispiel 10*

5 g Lysozym aus Hühnereiweiss werden in 200 ml H2O bei 80°C denaturiert, auf 38 °C abgekühlt, mit einem Enzymgemisch gemäss Beispiel hydrolysiert und wie in Beispiel beschrieben aufgearbeitet. Man erhält 3,4 g farbloses Produkt mit antimutagener und antiphlogistischer und lokal analgetischer Wirkung.

Das erfindungsgemässe Produkt wurde im Mäuseversuch auf folgende Weise pharmakologisch getestet.

Eine Pentoxyphyllin-Dosis von etwa 300 mg/kg, i.p. ist normalerweise tödlich für eine Maus. Verabreicht man jedoch etwa 30 Minuten vorher des erfindungsgemässe Produkt in einer Dosis von etwa 300 bis 600 mg/kg intraperitoneal oder intravenös, so erreicht man einen 60 bis 100%igen Schutzeffekt gegenüber der Pentoxyphyllin-induzierten Mortalität.

Die lokal analgetische Wirkung wurde auch am Auge des Menschen geprüft. Die Prüfung der antimutagenen Wirkung erfolgte am Hamster-Sister Chromatide Exchange Test.

*Beispiel 11*

5 g des Rohprodukts des Beispiel 1 werden mit 100 ml absolutem Äthanol bei einer Temperatur zwischen 22 und 50°C extrahiert. Man filtriert oder zentrifugiert und engt die erhaltene klare Lösung zur Trockene ein, dabei erhält man etwa 0,2 g Produkt (AP) als Rückstand.

Die analgetische Wirkung dieses Produktes ist so ausgeprägt, dass nur 1/8 der Menge des Produktes des Beispiels 1 benötigt wird, um den gleichen pharmakologischen Effekt zu erzielen.

*Beispiel 12*

100 g des nach Beispiel 11 erhaltenen Produktes (AP) werden in 500 ml 80%igem Äthanol suspendiert, zweimal mit jeweils 500 ml Heptan extrahiert. Die Heptanphase wird mit 200 ml 80%igem Äthanol nachgewaschen (Waschlösung wird mit Äthanolphase vereinigt). Anschliessend wird das Heptan abgezogen. Der Rückstand ist das Produkt (P), Ausbeute: 43 g. Durch Einengen der wässrig-alkoholischen Phase erhält man das Produkt (A2), Ausbeute: 54 g. Letzteres wird mit 1,5 l Diethyläther eine halbe Stunde heftig gerührt, man dekantiert die Ätherphase ab, isoliert den Rückstand und verrührt diesen heftig mit einem Liter Chloroform. Später wird die Chloroformphase abfiltriert, im Vakuum eingeengt und als Eindampfungsrückstand das Produkt (F) gewonnen, Ausbeute: 20 g.

Der chloroformlösliche Filterrückstand wird in 250 ml Ethanol aufgenommen und im Vakuum eingeengt. Man erhält so das Produkt (N); Ausbeute: 13 g. Durch Auflösen in 200 ml Chloroform und Einengen der Chloroformphase werden weitere 4 g an Produkt F erhalten. Die Ausbeute an Produkt (N) beträgt dann 8,5 g.

Auch das Produkt (F) kann zur völligen Entfernung von Chloroform-Resten in Ethanol aufgenommen und im Vakuum von allen flüchtigen Anteilen befreit werden.

Aus dem oben erhaltenen Produkt (P) kann durch Auflösen in absolutem Äthanol, Zugabe von Wasser bis zu einer bleibenden Trübung und anschliessendes Extrahieren mit Heptan weiteres Produkt (A$_2$) aus der ethanolischen Phase gewonnen werden (ca. 10% des eingesetzten Produktes P).

## Patentansprüche

1. Proteinhydrolysate aus Molkenprotein, Lactalbumin, α-Lactalbumin, Lactoferrin, β-Lactoglobulin, Lysozym oder Serumalbumin, dadurch erhältlich, dass man eine etwa 1-10 gew.%ige Suspension von Molkenprotein, Lactalbumin, α-Lactalbumin, Lactoferrin, β-Lactoglobulin, Lysozym oder Serumalbumin in Wasser bei 35 bis 38°C mehrere Stunden unter Rühren mit mindestens einer Protease und gegebenenfalls einer Lipase behandelt,

diese Behandlung bei etwa 60°C einige Stunden fortsetzt,

auf 80-90°C erwärmt, bei dieser Temperatur kurz verweilt, dann Abkühlen lässt,

nach dem Abkühlen unter reduziertem Druck einengt und den Rückstand als Produkt (A) isoliert;

mit einem polaren Lösungsmittel oder Lösungsmittelgemisch bei Raumtemperatur oder leicht erhöhter Temperatur extrahiert,

filtriert und aus dem Filtrat durch Einengen zur Trockene das Produkt (AP) gewinnt.

2. Verfahren zur Herstellung der Proteinhydrolysate gemäss Anspruch 1, dadurch gekennzeichnet, dass man eine etwa 1 bis 10 gew.%ige Suspension von Molkenprotein, Lactalbumin, α-Lactalbumin, Lactoferrin, β-Lactoglobulin, Lysozym oder Serumalbumin in Wasser bei 35 bis 38°C mehr als eine Stunde unter Rühren mit mindestens einer Protease und gegebenenfalls einer Lipase behandelt,

dann auf etwa 60°C erwärmt, diese Temperatur unter Rühren mehr als eine Stunde aufrechterhält, dann

auf 80-90°C erhitzt, kurze Zeit bei dieser Temperatur verweilt, abkühlt,

unter vermindertem Druck einengt und den Rückstand (Produkt A) isoliert,

letzteren Rückstand mit einem polaren Lösungsmittel oder Lösungsmittelgemisch bei Raumtemperatur oder leicht erhöhter Temperatur extrahiert, filtriert und

aus dem Filtrat durch Einengen zur Trockene das Produkt (AP) gewinnt.

3. Verfahren nach Anspruch 2, dadurch gekennzeichnet, dass man eine 2-5 gew.%ige Suspension des Ausgangsmaterials in Wasser verwendet.

4. Verfahren nach Anspruch 2 oder 3, dadurch gekennzeichnet, dass die Behandlung bei 35 bis 38°C etwa 1 bis 4 Stunden, vorzugsweise etwa 2 Stunden dauert und dass die erhöhte Temperatur von etwa 60°C etwa 1 bis 4, vorzugsweise etwa 2 bis 3 Stunden beibehalten wird.

5. Verfahren nach einem der Ansprüche 2 bis 4, dadurch gekennzeichnet, dass man als polares Lösungsmittel absolutes Äthanol, Chloroform oder Isopropanol bzw. als popolares Lösungsmittelgemisch ein Chloroform/Methanol-Gemisch (1/1 bis 3/1 Vol./Vol.) oder ein Äthanol/Wasser-Gemisch mit 20 bis 80% Wasser verwendet.

6. Verfahren nach einem der Ansprüche 2 bis 5, dadurch gekennzeichnet, dass man als Protease(n) Papain, Pankreatin, Chymotrypsin, Trypsin und gegebenenfalls eine aus Pilzen und/oder Bakterien gewonnene Protease verwendet, wobei die Pilzprotease ausgewählt wird unter den Proteasen aus Tritirachium-Arten, insbesondere Tritirachium alba, Proteasen aus Aspergillus-Arten, insbesondere Aspergillus saitoi, Aspergillus sojae, Aspergillus oryzae und/oder aus Rhizopus-Arten, insbesondere Newlase, und wobei die Bakterienprotease ausgewählt wird unter Proteasen aus Streptomyces-Arten, insbesondere Streptomyces caespitosus, Streptomyces griseus (Pronase E), aus Bacillus subtilis-Arten, insbesondere Subtilopeptidase A (Carlsberg Subtilisin) und aus Bacillus polymyxa.

7. Verfahren nach einem der Ansprüche 2 bis 6, dadurch gekennzeichnet, dass man die Protease(n) und/oder Lipase in einer Menge von etwa 0,01 bis 2 Gew.% bezogen auf die Suspension, verwendet.

8. Verfahren nach einem der Ansprüche 2 bis 7, dadurch gekennzeichnet, dass man als Protease Papain verwendet.

9. Verfahren nach einem der Ansprüche 2 bis 8, dadurch gekennzeichnet, dass man als Protease ein Gemisch von etwa gleichen Gew.-Teilen Pankreatin und Papain verwendet.

10. Verfahren nach einem der Ansprüche 2 bis 9, dadurch gekennzeichnet, dass man als Proteasen ein Gemisch von etwa gleichen Anteilen Papain, Pankreatin und einer Bakterien- oder Pilzprotease verwendet.

11. Verfahren nach einem der Ansprüche 2 bis 10, dadurch gekennzeichnet, dass man die wärmebeständigeren Enzyme in der zweiten Erwärmungsphase zugibt, das heisst nach dem ersten Erwärmen auf 35 bis 38°C.

12. Verfahren nach einem der Ansprüche 2 bis 11, dadurch gekennzeichnet, dass man

das Produkt (AP) in der etwa 10 bis 20fachen Gewichtsmenge eines 40 bis 80%igen wässrigen Äthanols suspendiert, mit etwa der gleichen Volumenmenge eines aliphatischen oder zykloaliphatischen Kohlenwasserstoffes mit etwa 4 bis 8 C-Atomen wiederholt extrahiert,

aus den einzelnen Phasen (der wässrig alkoholischen und dem Kohlenwasserstoffextrakt) jeweils das Lösungsmittel entfernt, wobei man als Rückstand der wässrig-alkoholischen Phase das Produkt (A$_2$) und als

Rückstand des Kohlenwasserstoffextraktes das Produkt (P) erhält;

das Produkt (A$_2$) mit der etwa 5 bis 20fachen Gewichtsmenge Diäthyläther extrahiert,

den ungelösten Anteil mit der etwa 20 bis 40fachen Gewichtsmenge Chloroform intensiv verrührt,

anschliessend filtriert,

das Filtrat zur Trockene einengt und damit das Produkt (F) isoliert und

als chloroformunlöslichen Anteil das Produkt (N) isoliert.

13. Pharmazeutische Mittel, enthaltend wenigstens ein Proteinhydrolysat gemäss Anspruch 1 bzw. erhältlich nach einem der Ansprüche 2 bis 12.

## Claims

1. Protein hydrolysate from lactic protein, lactalbumin, α-lactalbumin, lactoferrin, β-lactoglobulin, lysozyme or serum albumin, obtainable by treating a suspension of approximately 1-10% by weight of lactic protein, lactalbumin, α-lactalbumin, lactoferrin, β-lactoglobulin, lysozyme or serum albumin in water with at least one protease and optionally one lipase, stirring for several hours at a temperature of 35 to 38°C,

this treatment is continued for a few hours at a temperature of approximately 60°C,

heating to 80-90°C, maintaining at this temperature for a short time, and leaving to cool,

after cooling, concentrating under reduced pressure and isolating the residue as product (A):

extracting with a polar solvent or solvent mixture at room temperature or slightly above room temperature, and

filtering, the product (AP) being obtained from the filtrate by reducing to dryness.

2. Process for the manufacture of protein hydrolysate according to claim 1, characterised in that a suspension of 1 to 10% by weight of lactic protein, lactalbumin, α-lactalbumin, lactoferrin, β-lactoglobulin, lysozyme or serum albumin in water is treated with at least one protease and optionally one lipase, by stirring for more than 1 hour at a temperature of 35 to 38°C, then heated to approximately 60°C, this temperature being maintained for more than 1 hour whilst stirring,

then heated to 80 to 90°C, maintained at this temperature for a short time, and cooled,

concentrated under reduced pressure and the residue [product (A)] isolated,

the residue is extracted with a polar solvent or solvent mixture at room temperature or slightly above room temperature and filtered and

the product (AP) is obtained from the filtrate by reducing to dryness.

3. Process according to claim 2, characterised in that a 2 to 5% by weight suspension of the starting material in water is used.

4. Process according to claim 2 or 3, characterised in that the treatment lasts approximately 1 to 4 hours, preferably approximately 2 hours, at a temperature of 35 to 38°C and that the increased temperature of approximately 60°C is maintained for approximately 1 to 4 hours, preferably approximately 2 to 3 hours.

5. Process according to any one of claims 2 to 4, characterised in that as a polar solvent absolute ethanol, chloroform or isopropanol or as a polar solvent mixture a chloroform/methanol mixture (1/1 to 3/1 vol./vol.) or an ethanol/water mixture with 20 to 80% water is used.

6. Process according to any one of claims 2 to 5, characterised in that as protease(s) papain, pancreatin, chymotrypsin, trypsin and optionally a protease obtained from fungi and/or bacteria is used, whereby the fungal protease is selected from proteases of the Tritirachium species, especially Tritirachium alba, from proteases of the Aspergillus species, especially Aspergillus saitoi, Aspergillus sojae, Aspergillus oryzae and/or from Rhizopus species, especially Newlase, and whereby the bacterial protease is selected from proteases of the Streptomyces species, especially Streptomyces caespitosus, Streptomyces griseus (Pronase E), from Bacillus subtilis species, especially Subtilopeptidase A (Carlsberg subtilisin) and from Bacillus polymyxa.

7. Process according to any one of claims 2 to 6, characterised in that the protease(s) and/or lipase are used in an approximately 0.01 to 2% by weight suspension.

8. Process according to any one of claims 2 to 7, characterised in that papain is used as a protease.

9. Process according to any one of claims 2 to 8, characterised in that a mixture of approximately equal parts by weight of pancreatin and papain are used.

10. Process according to any one of claims 2 to 9, characterised in that a mixture of approximately equal parts of papain, pancreatin and a bacterial or fungal protease is used as proteases.

11. Process according to any one of claims 2 to 10, characterised in that the enzymes which are more resistant to heat are added in the second heating phase, i.e. after the first heating to 35 to 38°C.

12. Process according to any one of claims 2 to 11, characterised in that

the product (AP) is suspended in approximately 10 to 20 times its own weight of 40 to 80% aqueous ethanol,

repeatedly extracted with approximately the same volume of an aliphatic or cycloaliphatic hydrocarbon of approximately 4 to 8 C-atoms,

the solvent is removed from the respective phases (the aqueous alcoholic and the hydrocarbon extract), whilst obtaining

product ($A_2$) as residue of the aqueous-alcoholic phase and

product (P) as residue of the hydrocarbon extract;

product ($A_2$) is extracted with approximately 5 to 20 times its own weight, of diethyl ether

the undissolved part is vigorously mixed with approximately 20 to 40 times its own weight of chloroform

and filtered,

the filtrate is reduced to dryness and thus the product (F) isolated and

product (N) is isolated as the part insoluble in chloroform.

13. Pharmaceutical substance containing at least one protein hydrolysate according to claim 1 and obtainable according to any one of claims 2 to 12.

## Revendications

1. Hydrolysat de protéines constitués de protéine de petit-lait, de lactalbumine, d'α-lactalbumine, de lactoferrine, de β-lactoglobuline, de lysozyme ou d'albumine du sérum, pouvant être obtenu par un procédé dans lequel on traite une suspension à environ 1-10% en poids de protéine de petit-lait, de lactalbumine, d'α-lactalbumine, de lactorferrine, de β-lactoglobuline, de lysozyme ou d'albumine du sérum dans l'eau, à 35-38°C, pendant plusieurs heures sous agitation, avec au moins une protéase et éventuellement une lipase,

on poursuit ce traitement à environ 60°C pendant quelques heures,

on chauffe à 80-90°C, maintient à cette température pendant une courte durée, puis laisse refroidir,

après le refroidissement, on concentre sous pression réduite et isole le résidu comme produit (A);

on extrait avec un solvant ou un mélange de solvants polaires, à la température ambiante, ou à une température légèrement supérieure,

on filtre et à partir du filtrat, on obtient par concentration à sec, le produit (AP).

2. Procédé de préparation de l'hydrolysat de protéine selon la revendication 1, caractérisé en ce que l'on traite une suspension à environ 1-10% en poids de protéine de petit-lait, de lactalbumine, d'α-lactalbumine, de lactoferrine, de β-lactoglobuline, de lysozyme ou d'albumine du sérum dans l'eau, à 35-38°C, pendant plus d'une heure sous agitation, avec au moins une portéase et éventuellement une lipase,

ensuite on chauffe à environ 60°C, maintient cette température sous agitation pendant plus d'une heure, puis

on chauffe à 80-90°C, maintient pendant une courte durée à cette température, refroidit,

concentre sous pression réduite et isole le résidu qui est le produit (A),

extrait ce dernier résidu avec un solvant ou un mélange de solvants polaires à la température ambiante ou à une température légèrement supérieure, on filtre et

à partir du filtrat, par concentration à sec, on obtient le produit (AP).

3. Procédé selon la revendication 2, caractérisé en ce que l'on utilise une suspension aqueuse à 2-5% en poids du matériau de départ.

4. Procédé selon la revendication 2 ou 3, caractérisé en ce que le traitement à 35-38°C dure pendant environ 1 à 4 heures, de préférence environ 2 heures, et que la température d'environ 60°C est maintenue pendant eviron 1 à 4 heures de préférence pendant environ 2 à 3 heures.

5. Procédé selon l'une des revendications 2 à 4, caractérisé en ce que comme solvant polaire on utilise de l'éthanol absolu, du chloroforme ou de l'insopropanol, ou comme mélange de solvants polaires, on utilise un mélange chloroforme/méthanol (1/1 à 3/1 vol./vol.) ou un mélange éthanol/eau contenant 20 à 80% d'eau.

6. Procédé selon l'une des revendications 2 à 5, caractérisé en ce que comme protéase on utilise la papaïne, la pancréatine, la chymotrypsine, la trypsine et éventuellement une protéase obtenue à partir de champignons, et/ou de bactéries, la protéage fongique étant choisie parmi les protéases du genre Tritirachium, en particulier Tritirachium alba, les protésases du genre Aspergillus, en particulier Aspergillus saitoi, Aspergillus sojae, Aspergillus oryzae et/ou du genre Rhizopus, en particulier Newlase, et la protéase de bactéries étant choisie parmi les protéases du genre Streptomyces, en particulier Streptomyces caespitosus, Streptomyces griseus (pronase E), les protéases de Bacillus subtilis, en particulier la subtilo-peptidase A (Carlsberg Subtilisine) et les protéases de Bacillus polymyxa.

7. Procédé selon l'une des revendications 2 à 6, caractérisé en ce que le ou les protéases et/ou les lipases sont utilisés dans une proportion d'environ 0,01 à 2% en poids, par rapport à la suspension.

8. Procédé selon l'une des revendications 2 à 7, caractérisé en ce que comme protéase, on utilise la papaïne.

9. Procédé selon l'une des revendications 2 à 8, caractérisé en ce que comme protéase, on utilise un mélange en parties sensiblement égales en poids, de pancréatine et de papaïne.

10. Procédé selon l'une des revendications 2 à 9, caractérisé en ce que comme protéase, on utilise un mélange en parties sensiblement égales de papaïne, en pancréatine et d'un protéase bactérienne ou fongique.

11. Procédé selon l'une des revendications 2 à 10, caractérisé en ce que l'enzyme thermorésistante est introduite dans la deuxième étape de chauffage, c'est-à-dire après le premier chauffage à 35-38°C.

12. Procédé selon l'une des revendications 2 à 11, caractérisé en ce que

l'on met en suspension le produit (AP) dans environ 10 à 20 fois son poids d'éthanol aqueux à 40-80%,

on extrait de manière répétée avec environ un volume d'un hydrocarbure aliphatique ou cycloaliphatique, comprenant environ 4 à 8 atomes de carbone,

on élimine le solvant de chaque phase individuelle (l'extrait hydroalcoolique et l'extrait à l'hydrocarbure), et on obtient ainsi

comme résidu de la phase hydroalcoolique, le produit (A₂) et

comme résidu de l'extrait à l'hydrocarbure, le produit (P);

on extrait le produit (A₂) avec environ 5 à 20 fois son poids de diéthyl éther,

on agite intensément la partie non dissoute avec environ 20 à 40 fois son poids de chloroforme, ensuite on filtre,

concentre le filtrat à sec et isole ainsi le produit (F) et

isole le produit (N) qui est la fraction insoluble dans le chloroforme.

13. Composition pharmaceutique, contenant au moins un hydrolysat de protéine selon la revendication 1 ou qui peut être obtenu selon l'une des revendications 2 à 12.